Europäisches Patentamt

European Patent Office

Office européen des brevets

(19)

(11) **EP 0 583 490 B1**

(12) **EUROPÄISCHE PATENTSCHRIFT**

(45) Veröffentlichungstag und Bekanntmachung des Hinweises auf die Patenterteilung:
**22.04.1998  Patentblatt 1998/17**

(51) Int Cl.⁶: **G06F 19/00**

(21) Anmeldenummer: **92113112.4**

(22) Anmeldetag: **31.07.1992**

(54) **Verfahren zur Verarbeitung eines elektrischen, insbesondere von einem Herzen abgeleiteten Signals**

Procedure to process an electrical signal, especially a signal derived from the heart

Procédé de traitement d'un signal électrique, en particulier un signal dérivé du coeur

(84) Benannte Vertragsstaaten:
**DE FR GB IT NL SE**

(43) Veröffentlichungstag der Anmeldung:
**23.02.1994   Patentblatt 1994/08**

(73) Patentinhaber: **Pacesetter AB**
**171 95 Solna (SE)**

(72) Erfinder: **Högnelid, Kurt**
**S-137 38 Västerhaninge (SE)**

(74) Vertreter: **Lettström, Richard Wilhelm**
**H. Albihns Patentbyra AB,**
**Box 3137**
**103 62 Stockholm (SE)**

(56) Entgegenhaltungen:
**GB-A- 2 123 560**

- **BIOMEDIZINISCHE TECHNIK Bd. 25, Nr. 1/2, Januar 1980, BERLIN, DE Seiten 2 - 6 W KRAFT ET AL 'EKG-DATENREDUKTION MITTELS EINES MIKROCOMPUTERS'**
- **PROCEEDINGS OF THE ANNUAL INTERNATIONAL CONFERENCE OF THE IEEE ENGINEERING IN MEDICINE AND BIOLOGY SOCIETY, IEEE PRESS, NEW YORK, US Bd. 1/4, 4. November 1988, NEW ORLEANS, LOUSIANA, US Seiten 149 - 150 , XP000118249 M.L.HEIMER ET AL 'BIOSIGNAL RECOGNITION USING SERIAL DATA FROM A DELTA MODULATOR'**
- **IEEE TRANSACTIONS ON BIOMEDICAL ENGINEERING, IEEE PRESS Bd. 35, Nr. 6, Juni 1988, NEW YORK, US Seiten 489 - 494 , XP000051052 BORIVOJE FURTH ET AL 'AN ADAPTIVE REAL TIME ECG COMPRESSION ALGORITHM WITH VARIABLE THRESHOLD'**

**Beschreibung**

Die Erfindung betrifft ein Verfahren zur Verarbeitung eines elektrischen, insbesondere von einem Herzen abgeleiteten Signals, wobei das elektrische Signal mittels Deltamodulation in ein binäres Vorzeichensignal bestehend aus einer Impulsfolge mit den zeitdiskreten logischen Vorzeichenwerten Null oder Eins umgesetzt wird.

Bei derartigen, beispielsweise aus der EP-A-0 402 508 oder der US-A-4 567 883 bekannten Verfahren wird ein von einem Herzen abgeleitetes elektrisches Signal in einem Deltamodulator mit einem entweder eine konstante positive oder eine konstante negative Steigung aufweisenden sogenannten Vorhersagesignal oder Prädiktorsignal verglichen und am Ausgang des Deltamodulators ein binäres Vorzeichensignal mit den Vorzeichenwerten Eins und Null erzeugt, je nachdem, ob das elektrische Signal das Vorhersagesignal über- oder unterschreitet. Bei jedem Vorzeichenwechsel von Eins auf Null erfolgt eine Änderung des Vorhersagesignals von der positiven Steigung auf die negative Steigung, während bei einem Vorzeichenwechsel von Null auf Eins das Vorzeichensignal in umgekehrter Weise geändert wird. Das binäre Vorzeichensignal am Ausgang des Deltamodulators gibt also an, inwieweit das elektrische Signal dem Vorhersagesignal mit den zwei unterschiedlichen konstanten Steigungen folgt. Um eine bessere Anpassung des Vorhersagesignals an den Verlauf des elektrischen Signals zu erhalten und damit das Modulationsrauschen, also die Fehlerdifferenz zwischen dem elektrischen Signal und dem Vorhersagesignal möglichst gering zu halten, ist bei dem aus der US-A-4 567 883 bekannten Verfahren zusätzlich vorgesehen, daß in Abhängigkeit von dem Vorzeichensignal am Ausgang des Deltamodulators unterschiedliche Beträge für die Steigung des Vorhersagesignals einstellbar sind.

Für eine weitere Auswertung des deltamodulierten Signals ist es oft wünschenswert, die Impulsfolge des binären Vorzeichensignals zunächst in einem Speicher abzuspeichern und bei Bedarf für die Signalsauswertung aus dem Speicher abzurufen. Ein Beispiel hierfür ist die Abspeicherung eines durch Deltamodulation digitalisierten intrakardiellen Elektrogramms in einem Speicher eines implantierbaren Herzschrittmachers oder Defibrillators, um das abgespeicherte intrakardielle Elektrogramm zu einem späteren Zeitpunkt für diagnostische oder therapeutische Zwecke heranziehen zu können. Damit eine sinnvolle Auswertung des intrakardiellen Elektrogramms möglich ist, muß dieses zumeist über einen längeren Zeitraum erfaßt und abgespeichert werden, so daß eine sehr große abzuspeichernde Datenmenge anfällt. Andererseits ist aber die in implantierbaren Geräten realisierbare Speicherkapazität auf Grund der geringen Baugröße und des kompakten Aufbaus solcher Geräte begrenzt.

Der Erfindung liegt daher die Aufgabe zugrunde, ein Verfahren zur Komprimierung eines deltamodulierten elektrischen, insbesondere von einem Herzen abgeleiteten Signals anzugeben.

Die Erfindung wird definiert in Anspruch 1.

Gemäß der Erfindung wird die Aufgabe dadurch gelöst, daß bei dem Verfahren der eingangs angegebenen Art die Impulsfolge in vier unterschiedliche Datenwörter umgesetzt wird, die jeweils unmittelbar aufeinanderfolgend in Reihenfolge des Auftretens von vier möglichen Zuständen erzeugt werden, wobei ein erstes Datenwort eine erste Zustandskennung und die Länge eines ersten Zustands angibt, bei dem das Vorzeichensignal für eine vorgegebene erste Mindestdauer kontinuierlich den logischen Wert Null aufweist, ein zweites Datenwort eine zweite Zustandskennung und die Länge eines zweiten Zustands angibt, bei dem das Vorzeichensignal für eine vorgegebene zweite Mindestdauer kontinuierlich den logischen Wert Eins aufweist, ein drittes Datenwort eine dritte Zustandskennung und die Länge eines dritten Zustands abgibt, bei dem der Vorzeichenwert innerhalb der vorgegebenen Mindestdauern wechselt und die Summe aller Vorzeichenwerte Eins die Summe aller Vorzeichenwerte Null um einen vorgegebenen ersten Betrag überschreitet, und ein viertes Datenwort eine vierte Zustandskennung und die Länge eines vierten Zustands angibt, bei dem der Vorzeichenwert innerhalb der vorgegebenen Mindestdauern wechselt und die Summe aller Vorzeichenwerte Null die Summe aller Vorzeichenwerte Eins um einen vorgegebenen zweiten Betrag überschreitet.

Der Verlauf des elektrischen Signals wird also mit Hilfe der vier Datenwörter als eine Folge von vier unterschiedlichen Zuständen beschrieben, die jeweils eine unterschiedliche Länge aufweisen können. Dabei ist der erste Zustand dadurch chakterisiert, daß das elektrische Signal während einer längeren Zeitdauer das positiv ansteigende Vorhersagesignal des Deltamodulators übersteigt, also eine bestimmte positve Mindeststeilheit aufweist, während bei dem zweiten Zustand das elektrische Signal ebenfalls während einer längeren Zeitdauer das negativ abfallende Vorhersagesignal unterschreitet, also eine bestimmte negative Mindeststeilheit aufweist. Bei einem flacheren Verlauf des elektrischen Signals gibt der dritte Zustand an, daß das elektrische Signal während der Dauer dieses Zustands im Mittel um den ersten vorgegebenen Betrag angestiegen ist, während bei dem vierten Zustand das elektrische Signal im Mittel um den vorgegebenen zweiten Betrag abgesunken ist.

Zur Vereinfachung des erfindungsgemäßen Verfahrens und damit zur Verringerung des zur Durchführung des Verfahrens erforderlichen Rechen- bzw. Hardware-Aufwands sind vorzugsweise für die erste und zweite Mindestdauer gleiche Werte vorgesehen.

In entsprechender Weise können auch für den ersten und zweiten Betrag gleiche Werte vorgesehen sein.

Die Länge der jeweiligen Zustände wird auf einfachste Weise dadurch bestimmt, daß die Anzahl der während dieser Zustände auftretenden zeitdiskreten Vorzeichenwerte in einem Zähler gezählt wird.

Wegen der sowohl programmäßig als auch hardwaremäßig einfachen Realisierbarkeit von Zählfunktionen ist entsprechend einer weiteren vorteilhaften Ausbildung des erfindungsgemäßen Verfahrens vorgesehen, daß zur Bestimmung, ob die Summe der Vorzeichenwerte Eins die Summe der Vorzeichenwerte Null um die vorgegebenen Beträge über- bzw. unterschreitet, das Auftreten der Vorzeichenwerte in einem weiteren Zähler mit in Abhängigkeit von den jeweiligen Vorzeichenwerten Null oder Eins umschaltbarer Zählrichtung gezählt wird, bis der Zählwert einen der vorgegebenen Beträge über- bzw. unterschreitet.

Wie eingangs bereits im Zusammenhang mit der US-A-4 567 883 erläutert, kann bei der Deltamodultion, bei der das Vorzeichensignal in Abhängigkeit davon erzeugt wird, ob das elektrische Signal ein mit einem vorgegebenen Änderungsbetrag in unterschiedlichen Richtungen änderbares Vorhersagesignal (Prädiktorsignal) über- oder unterschreitet, das Modulationsrauschen dadurch verhindert werden, daß unterschiedliche Änderungsbeträge für das Vorhersagesignal in Abhängigkeit von dem Vorzeichensignal einstellbar sind und daß die Datenwörter zusätzlich jeweils einen den aktuell eingestellten Änderungsbetrag bezeichnenden Informationsanteil enthalten.

Zur Erläuterung der Erfindung wird im folgenden auf die Figuren der Zeichnung Bezug genommen. Im einzelnen zeigen

FIG 1   ein Ausführungsbeispiel für einen Deltamodulator,

FIG 2   in einem Diagramm ein Beispiel für ein dem Deltamodulator zugeführtes elektrisches Signal und das von dem Deltamodulator ausgangsseitig erzeugte binäre Vorzeichensignal,

FIG 3   ein Ausführungsbeispiel für eine Schaltungsanordnung zur Realisierung des erfindungsgemäßen Verfahrens und

FIG 4   in einem Diagramm ein weiteres Beispiel für das elektrische Signal und dessen Umsetzung in Datenwörter.

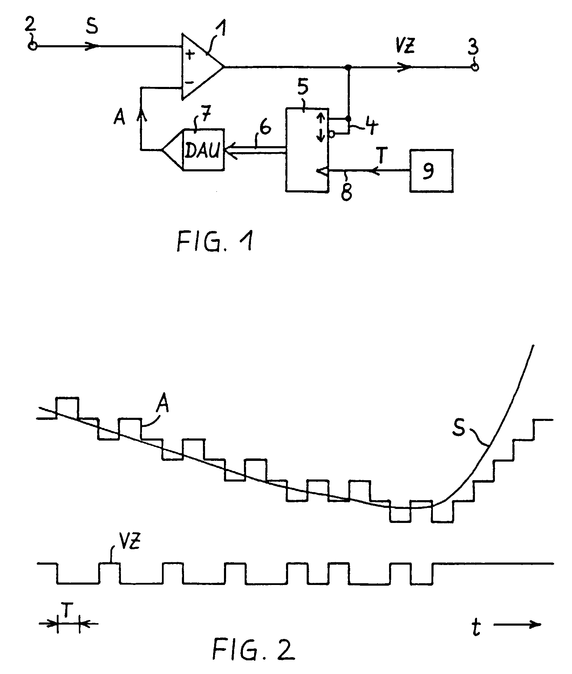

Figur 1 zeigt ein einfaches Ausführungsbeispiel für einen Deltamodulator mit einem Komparator 1, der über einen ersten, nichtinvertierenden Eingang (+) mit einem Eingang 2 des Deltamodulators für ein in ein binäres Vorzeichensignal VZ umzusetzendes elektrisches Signal S, beispielsweise ein Elektrokardiogramm verbunden ist. Der Ausgang 3 des Komparators 1 ist mit einem Steuereingang 4 einer Zähleinrichtung 5 verbunden, deren Ausgang 6 über einen Digital-/Analog- Umsetzer 7 mit einem zweiten invertierenden Eingang (-) des Komparators 1 verbunden ist. Die Zähleinrichtung 5 ist über einen Takteingang 8 an einem Taktsignalgeber 9 angeschlossen.

Der Komparator 1 nimmt an seinem Ausgang 3 jedesmal dann den logischen Zustand Eins an, wenn das elektrische Signal S an dem nichtinvertierenden Eingang (+) den Ausgangswert A des Digital-/Analog- Umsetzers 7 übersteigt. Fällt dagegen das elektrische Signal S unter den Ausgangswert A des Digital-/Analog-Umsetzers 7, so nimmt der Ausgang 3 des Komparators 1 den logischen Zustand Null an. Die Zähleinrichtung 5 zählt fortlaufend den Takt des von dem Taktsignalgeber 9 erzeugten Taktsignals T, wobei die Zählrichtung über den Steuergang 4 in Abhängigkeit von dem jeweiligen logischen Zustand am Ausgang 3 des Komparators 1 in der Weise gesteuert wird, daß die Zähleinrichtung 5 aufwärts zählt, wenn der logische Zustand den Wert Eins aufweist und abwärts zählt, wenn der logische Zustand Null beträgt. Auf diese Weise erzeugt die Zähleinrichtung 5 an ihrem Ausgang 6 ein digitales Zählsignal, das nach seiner Umsetzung in das analoge Vorhersagesignal A in dem Komparator 1 mit dem elektrischen Signal S verglichen wird und je nach Abweichung von dem elektrischen Signal S über die Zähleinrichtung 5 entweder nach oben oder nach unten an das elektrische Signal S angeglichen wird. Das Zählsignal bzw. das dazu analoge Vorhersagesignal A folgt dabei mit einem stufenförmigen Verlauf dem elektrischen Signal S wobei die Stufenhöhe durch das Zählinkrement und die Stufenbreite durch den Takt der Taktsignals T vorgegeben ist. Das Vorzeichensignal VZ am Ausgang 3 des Komparators 1 gibt die aktuelle Zählrichtung und damit die Richtung der Anpassung des analogen Vorhersagesignals A an das elektrische Signal S an.

Figur 2 zeigt ein Beispiel für den Verlauf des elektrischen Signals S, das von dem Digital-/Analog- Umsetzer 7 erzeugte analoge Vorhersagesignal A und das Vorzeichensignal VZ am Ausgang 3 des Komparators 1.

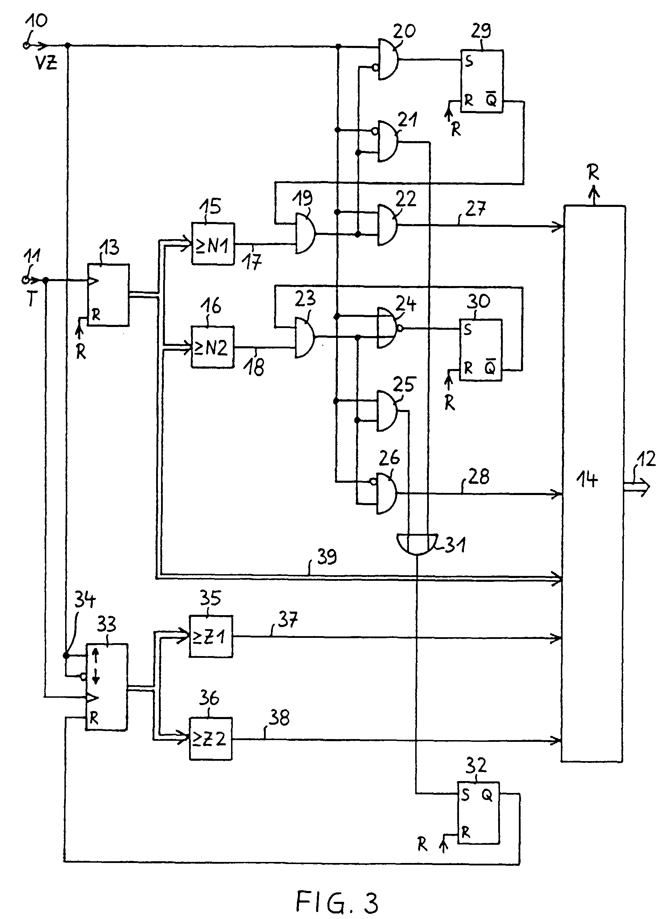

Figur 3 zeigt eine Schaltungsanordnung zur Realisierung des erfindungsgemäßen Verfahrens. Die Schaltungsanordnung weist einen ersten Eingang 10 für das von dem Ausgang 3 des Deltamodulators nach Figur 1 kommende Vorzeichensignal VZ, einen zweiten Eingang 11 für das von dem Taktsignalgenerator 9 kommende und das Zeitraster für die logischen Vorzeichenwerte Null und Eins des Vorzeichensignals VZ vorgebende Taktsignal T und einen Ausgang 12 auf, über den das zu Datenwörtern komprimierte Vorzeichensignal VZ abgegeben wird. Das Taktsignal T wird einem Zähler 13 zugeführt, der über einen Steuerausgang R einer Steuereinrichtung 14 auf einen anfänglichen Zählerstand Null zurücksetzbar ist, und entsprechend dem Takt des Taktsignals T seinen Zählerstand inkrementiert. Der Zählerstand des Zählers 13 wird einem ersten Zählerstandsvergleicher 15 und einem zweiten Zählerstandsvergleicher 16 zugeführt, von denen der Zählerstandsvergleicher 15 an seinem Ausgang 17 ein Ausgangssignal, also den logischen Ausgangszustand Eins, erzeugt, wenn der Zählerstand einen vorgegebenen Vergleichswert N1 überschreitet, und der zweite Zählerstandsvergleicher 16 an seinem Ausgang 18 ein Ausgangssignal erzeugt, wenn der Zählerstand einen weiteren vorgegebenen Vergleichswert N2 überschreitet. Der Ausgang 17 des ersten Zählerstandsvergleichers 15 ist über ein UND-Glied 19 mit jeweils einem Eingang von drei logischen Gliedern 20,21 und 22 verbunden, deren zweite Eingänge mit dem Vorzeichensignal VZ beaufschlagt sind. Der Ausgang 18 des zweiten Zählerstandsvergleichers 16 ist über ein weiteres UND-Glied 23 mit jeweils einem Eingang von drei weiteren logischen Gliedern 24,25 und 26 verbunden, deren zweite Eingänge ebenfalls mit dem Vorzeichensignal VZ beaufschlagt sind. Im einzelnen erzeugen die logischen Glieder 20-22 und 24-26 unter folgenden Bedingungen jeweils ein Ausgangssignal. Das logische Glied 20 erzeugt dann ein Ausgangssignal, wenn das Vorzeichensignal VZ den Wert Eins annimmt, bevor der Zählerstand des Zählers 13 den ersten Vergleichswert N1 erreicht hat. Das logische Glied 21 erzeugt dann ein Ausgangssignal, wenn das Vorzeichensignal VZ den Wert Null aufweist und der Zählerstand den Vergleichswert N1 erreicht oder überschritten hat. Das logische Glied 22 erzeugt dann ein Ausgangssignal, wenn der Zählerstand den ersten Vergleichswert N1 erreicht oder überschritten hat und das Vorzeichensignal VZ den Wert Eins annimmt. Das logische Glied 24 erzeugt dann ein Ausgangssignal, wenn das Vorzeichensignal VZ den Wert Null annimmt, bevor der Zählerstand den zweiten Vergleichswert N2 erreicht hat. Das logische Glied 24 erzeugt dann ein Ausgangssignal, wenn das Vorzeichensignal VZ den Wert Eins aufweist und der Zählerstand den zweiten Vergleichswert N2 erreicht oder überschritten hat. Schließlich erzeugt das logische Glied 26 dann ein Ausganssignal, wenn der Zählerstand den zweiten Vergleichswert N2 erreicht oder überschritten hat und das Vorzeichensignal VZ den Wert Null annimmt.

Die logischen Glieder 22 und 26 sind ausgangsseitig jeweils über Steuerleitungen 27 bzw. 28 mit der Steuereinrichtung 14 verbunden. Das logische Glied 20 ist ausgangsseitig mit dem Setzeingang S eines ersten Flip-Flops 29 verbunden, das an seinem invertierenden Ausgang $\bar{Q}$ mit einem weiteren Eingang des dem erstem Zählerstandsvergleicher 15 nachgeordneten UND-Glieds 19 verbunden ist. Das logische Glied 24 ist in entsprechender Weise ausgangsseitig mit dem Setzeingang S eines zweiten Flip-Flops 30 verbunden, das an seinem invertierenden Ausgang $\bar{Q}$ mit einem weiteren Eingang des dem zweiten Zählerstandsvergleichers 16 nachageordneten UND-Glieds 23 verbunden ist. Die logischen Glieder 21 und 25 sind ausgangsseitig über ein ODER-Glied 31 mit dem Setzeingang S eines dritten Flip-Flops 32 verbunden, dessen nichtinvertierender Ausgang Q mit dem Rücksetzeingang R eines weiteren Zählers 33 verbunden ist. Die Flip-Flops 29,30 und 32 sind über den Steuerausgang R der Steuereinrichtung 14 zurücksetzbar. Der weitere Zähler 33 weist einen mit dem Taktsignal T beaufschlagten Zähleingang und einen mit dem Vorzeichensignal VZ beaufschlagten Steuereingang 34 auf, über den die Zählrichtung in der Weise umschaltbar ist, daß der Zähler 33 bei einem Vorzeichenwert Eins aufwärts und bei einem Vorzeichenwert Null abwärts zählt. Der jeweilige Zählerstand des weiteren Zählers 33 ist einem dritten Zählerstandsvergleicher 35 und einem vierten Zählerstandsvergleicher 36 zugeführt, von denen der dritte Zählerstandsvergleicher 35 an seinem Ausgang 37 ein Ausgangssignal erzeugt, wenn der Zählerstand einen dritten vorgegebenen Vergleichswert Z1 überschreitet und der vierte Zählerstandsvergleicher an seinem Ausgang 38 ein Ausgangssignal erzeugt, wenn der Zählerstand einen vorgegebenen vierten negativen Vergleichswert -Z2 unterschreitet. Die Ausgänge 37 und 38 der beiden Zählerstandsvergleicher 35 und 36 sind jeweils mit der Steuereinrichtung 14 verbunden. Schließlich ist der Ausgang des ersten Zählers 13 über eine Steuerleitung 39 ebenfalls mit der Steuereinrichtung 14 verbunden.

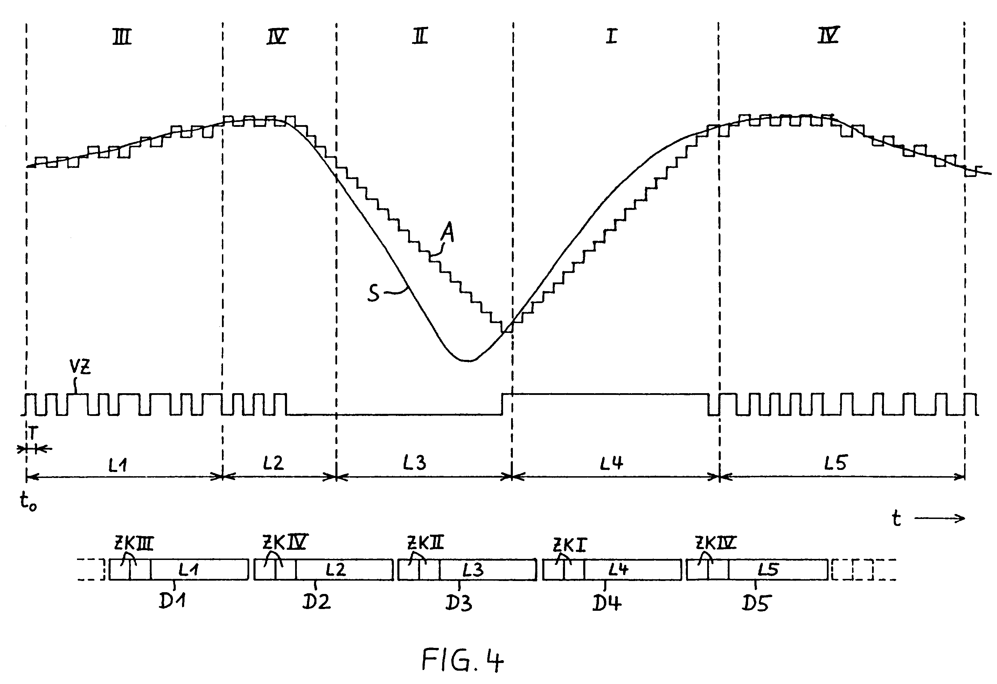

Zur Erläuterung der Funktionsweise der Schaltungsanordnung nach Figur 3 wird im folgenden auf die Figur 4 Bezug genommen, die als Beispiel für das elektrische Signal S einen QRS-Komplex aus einem Elektrokardiogramm zeigt. Ferner sind das Vorhersagesignal A am Ausgang des Digital/Analog- Umsetzers 7 (Figur 1) und das Vorzeichensignal VZ dargestellt.

Zu einem Startzeitpunkt $t_0$ sind die Zähler 13 und 33 und die Flip-Flops 29,30 und 32 zurückgesetzt. Für das beschriebene Beispiel wird angenommen, daß die Vergleichswerte N1,N2,Z1 und Z2 alle jeweils den gleichen Betrag "4" aufweisen. Bei dem ersten Takt des Taktsignals T übersteigt das elektrische Signal S das Vorhersagesignal A, so daß das Vorzeichensignal VZ den logischen Wert Eins aufweist. Hieraus ergibt sich die Annahme, daß das elektrische Signal S im folgenden ansteigt. Da der durch das Taktsignal T inkrementierte Zähler 13 noch nicht den Zählwert N1 = 4 erreicht hat, wird über das logische Glied 20 das Flip-Flop 29 gesetzt, daß über seinen invertierenden Ausgang $\bar{Q}$ und das UND-Glied 19 den Ausgang 17 des ersten Zählerstandvergleichers 15 sperrt.

Auf Grund des Vorzeichenwerts Eins des Vorzeichensignals VZ wird das Vorhersagesignal A beim nächsten Takt um eine Stufe erhöht, wobei das Vorhersagesignal A das elektrische Signal S übersteigt und eine Änderung des Vorzeichensignals VZ auf den Vorzeichenwert Null bewirkt. Da der Zähler 13 noch nicht den Zählwert N2 = 4 erreicht hat, wird über das logische Glied 24 das Flip-Flop 30 gesetzt, das über seinen invertierenden Ausgang $\bar{Q}$ und das UND-Glied 23 den Ausgang 18 des zweiten Zählerstandvergleichers 16 sperrt. Mit der Sperrung der Ausgänge 17 und 18 der beiden Zählerstandsvergleicher 15 und 16 wird dem Umstand Rechnung getragen, daß das Vorzeichensignal vor dem Erreichen der Zählwerte N1 = N2 = 4 seinen Vorzeichenwert gewechselt hat und daß deswegen ausgeschlossen ist, daß das elektrische Signal S für eine längere Zeitdauer einen steileren Verlauf aufweist als die aufgrund der Stufenhöhe maximal mögliche positive oder negative Steigung des Vorhersagesignals A.

Vom ersten Takt an wird der Zählerstand des weiteren Zählers 33 bei jedem Vorzeichenwert Eins inkrementiert und bei jedem Vorzeichenwert Null dekrementiert. Nach insgesamt 19 Takten erreicht dabei der Zählerstand des weiteren Zählers 33 den Vergleichswert ZI = 4, so daß der dritte Zählerstandsvergleicher 35 über seinen Ausgang 37 ein Steuersignal an die Steuereinrichtung 14 abgibt. Diese ruft daraufhin den Zählerstand des Zählers 13 ab, der den Wert "19" aufweist und damit die Länge L1 eines Zustands III bezeichnet, während dessen das elektrische Signal S mit einer im Durchschnitt geringeren Steigung als die maximal mögliche Steilheit des Vorhersagesignals A insgesamt um den Betrag Z1 = 4 angestiegen ist. Zur Angabe dieses Zustands III erzeugt die Steuereinrichtung 14 an ihrem Ausgang 12 ein Datenwort D1, das an seinen beiden ersten Bit-Stellen eine Zustandskennung ZKIII für den Zustand III angibt und mit den übrigen Bit-Stellen die Länge L1 des Zustands III bezeichnet.

Gleichzeitig mit der Erzeugung des Datenworts D1 werden die Zähler 13 und 33 und die Flip-Flops 29,30 und 32 durch die Steuereinrichtung 14 zurückgesetzt. Auch bei den folgenden Takten des Taktsignals T ändert sich der Vorzeichenwert des Vorzeichensignals VZ bevor der Zählerstand des Zählers 13 einen der beiden Vergleichswerte N1 oder N2 erreicht. Nach diesmal insgesamt 11 Takten erreicht der Zählerstand des weiteren Zählers 33 den Vergleichswert -Z2 des vierten Zählerstandsvergleichers 36, so daß dieser an seinem Ausgang 38 ein Signal an die Steuereinrichtung 14 abgibt. Diese ruft daraufhin wiederum den Zählerstand des Zählers 13 ab, der den Wert "11" aufweist und damit die Länge L2 eines Zustands IV bezeichnet, währenddessen das elektrische Signal S mit einer im Durchschnitt geringeren Neigung als die maximal mögliche negative Steilheit des Vorhersagesignals A um den Betrag Z2 = 4 abgesunken ist. Die Steuereinrichtung 14 erzeugt daraufhin ein neues Datenwort D2, das an seinen beiden ersten Bit-Stellen eine Zustandskennung ZKIV für den Zustand IV enthält und mit den übrigen Bit-Stellen die Länge L2 des Zustands IV angibt.

Gleichzeitig mit der Erzeugung des Datenworts D2 werden die Zähler 13 und 33 und die Flip-Flops 29,30 und 32 durch die Steuereinrichtung 14 zurückgesetzt. Bei dem ersten nachfolgenden Takt des Taktsignals T unterschreitet das elektrische Signal S das Vorhersagesignal A, so daß das Vorzeichensignal VZ den logischen Wert Null aufweist. Als Folge davon wird der Ausgang 18 des zweiten Zählerstandsvergleichers 16 über das logische Glied 24, das Flip-Flop 30 und das UND-Glied 23 gesperrt. Der Vorzeichenwert des Vorzeichensignals VZ ändert sich auch während der folgenden Takte nicht, so daß der Zählerstand des ersten Zählers 13 nach vier Takten den Vergleichswert N1 des ersten Zählerstandsvergleichers 15 erreicht. Dieser erzeugt daraufhin an seinem Ausgang 17 ein Ausgangssignal, mit dem über das logische Glied 21 und das ODER-Glied 31 das weitere Flip-Flop 32 gesetzt wird, welches über seinen nichtinvertierenden Ausgang Q den zweiten Zähler 33 zurücksetzt. In dem ersten Zähler 13 werden die Takte des Taktsignals T weiterhin solange gezählt, bis das Vorzeichensignal VZ seinen Vorzeichenwert von Null auf Eins ändert. Diese Änderung wird von dem logischen Glied 22 detektiert und über die Steuerleitung 27 der Steuereinrichtung 14 gemeldet. Diese ruft daraufhin den Zählerstand des Zählers 13 ab, der bei dem gezeigten Ausführungsbeispiel den Wert "17" aufweist und damit die Länge L3 eines Zustands II bezeichnet, während dessen das elektrische Signal S mit einer im Durchschnitt größeren Neigung als die Steilheit der Stufen des Vorhersagesignals A abfällt. Die steuerbare Schalteinrichtung 14 erzeugt an ihren Ausgang 12 daraufhin ein Datenwort D3, daß an seinen beiden ersten Bit-Stellen eine Zustandskennung ZKII für den Zustand II enthält und mit den übrigen Bit-Stellen die Länge L3 des Zustands II angibt.

Geichzeitig mit dem Erzeugen des Datenworts D3 werden die Zähler 13 und 33 und die Flip-Flops 29,30 und 32 durch die Steuereinrichtung 14 zurückgesetzt. Während der folgenden Takte des Taktsignals T übersteigt das elektrische Signal S das Vorhersagesignal A, bis bei dem 20. Takt ein Wechsel des Vorzeichensignals VZ von dem Vorzeichenwert Eins auf den Vorzeichenwert Null erfolgt. Dieser Vorzeichenwechsel wird von dem logischen Glied 26 detektiert und der Steuereinrichtung 14 über die Steuerleitung 28 mitgeteilt. Die Steuereinrichtung 14 ruft daraufhin den Zählerstand des Zählers 13 ab, der den Wert "20" aufweist und damit die Länge L4 eines Zustands I bezeichnet, während dessen das elektrische Signal S eine im Durchschnitt größere Steigung als die Steilheit der Stufen des Vorhersagesignals A aufweist. Die Steuereinrichtung 14 erzeugt an ihrem Ausgang 12 ein Datenwort D4, das an seinen beiden ersten Bit-Stellen eine Zustandskennung ZKI für den Zustand I enthält und an seinen übrigen Bit-Stellen die Länge L4 des Zustands I angibt.

Im Anschluß an den Zustand I tritt wieder ein Zustand IV auf, bei dem das elektische Signal S einen vergleichsweise flachen Verlauf aufweist und während der Dauer L5 dieses Zustands IV um den Betrag -Z2 absinkt. Dieser neue Zustand IV wird durch ein Datenwort D5 bezeichnet, daß an seinen ersten beiden Bit-Stellen wiederum eine Zustandskennung ZKIV für den Zustand IV enthält und mit den übrigen Bit-Stellen die Länge L5 dieses Zustands IV bezeichnet.

Durch das angegebene Verfahren wird also das Vorzeichensignal VZ am Ausgang des Deltamodulators zu Datenwörtern D1-D5 komprimiert, die jeweils einen der vier möglichen Zustände I-IV und seine Länge L1-L4 bezeichnen. Während der Zustände I und II ist die mittlere Steigung des elektrischen Signals S größer als die maximal mögliche positive bzw. negative Steigung des Vorhersagesignals A, während bei den Zuständen III und IV das elektrische Signal S eine geringere mittlere Steilheit aufweist und dabei insgesamt um den Betrag Z1 ansteigt bzw. um den Betrag Z2 abfällt.

Die Schaltungsanordnung in Figur 3 ist lediglich als ein Beispiel zur Durchführung des erfindungsgemäßen Verfahrens anzusehen, das auch durch den Ablauf eines Programms realisiert werden kann.

**Patentansprüche**

1. Verfahren zur Verarbeitung eines elektrischen, insbesondere von einem Herzen abgeleiteten Signals (S), wobei das elektrische Signal (S) mittels Deltamodulation in ein binäres Vorzeichensignal (VZ) bestehend aus einer Impulsfolge mit den zeitdiskreten logischen Vorzeichenwerten Null oder Eins umgesetzt wird, **dadurch gekennzeichnet**, daß die Impulsfolge in vier unterschiedliche Datenwörter (D1-D4) umgesetzt wird, die jeweils unmittelbar aufeinanderfolgend in der Reihenfolge des Auftretens von vier möglichen Zuständen (I-IV) erzeugt werden, wobei

ein erstes Datenwort (D4) eine erste Zustandskennung (ZKI) und die Länge (L4) eines ersten Zustands (I) angibt, bei dem das Vorzeichensignal (VZ) für eine vorgegebene erste Mindestdauer (N1) kontinuierlich den logischen Wert Null aufweist,
ein zweites Datenwort (D3) eine zweite Zustandskennung (ZKII) und die Länge (L3) eines zweiten Zustands (II) angibt, bei dem das Vorzeichensignal (VZ) für eine vorgegebene zweite Mindestdauer (N2) kontinuierlich den logischen Wert Eins aufweist,
ein drittes Datenwort (D1) eine dritte Zustandskennung (ZKIII) und die Länge (L1) eines dritten Zustands (III) angibt, bei dem der Vorzeichenwert innerhalb der vorgegebenen Mindestdauern (N1, N2) wechselt und die Summe aller Vorzeichenwerte Eins die Summe aller Vorzeichenwerte Null um einen vorgegebenen ersten Betrag (Z1) überschreitet, und
ein viertes Datenwort (D2) eine vierte Zustandskennung (ZKIV) und die Länge (L2) eines vierten Zustands (IV) angibt, bei dem der Vorzeichenwert innerhalb der vorgegebenen Mindestdauern (N1, N2) wechselt und die Summe aller Vorzeichenwerte Null die Summe aller Vorzeichenwerte Eins um einen vorgegebenen zweiten Betrag (Z2) überschreitet.

2. Verfahren nach Anspruch 1, **dadurch gekennzeichnet**, daß für die erste und zweite Mindestdauer (N1, N2) gleiche Werte vorgesehen sind.

3. Verfahren nach Anspruch 1 oder 2, **dadurch gekennzeichnet**, daß für den ersten und zweiten Betrag (Z1, Z2) gleiche Werte vorgesehen sind.

4. Verfahren nach einem der vorangehenden Anprüche, **dadurch gekennzeichnet**, daß zur Bestimmung der Länge (L1-L4) der jeweiligen Zustände (I-IV) die Anzahl der während dieser Zustände (I-IV) auftretenden zeitdiskreten Vorzeichenwerte in einem Zähler (13) gezählt wird.

5. Verfahren nach einem der vorangehenden Ansprüche, **dadurch gekennzeichnet**, daß zur Bestimmung, ob die Summe aller Vorzeichenwerte Eins die Summe aller Vorzeichenwerte Null um die vorgegebenen Beträge (Z1, Z2) über- bzw. unterschreitet, das Auftreten der Vorzeichenwerte in einem weiteren Zähler (33) mit in Abhängigkeit von den jeweiligen Vorzeichenwerten Null oder Eins umschaltbarer Zählrichtung gezählt wird, bis der Zählwert einen der vorgegebenen Beträge (Z1 oder Z2) über- bzw. unterschreitet.

6. Verfahren nach einem der vorangehenden Ansprüche, **dadurch gekennzeichnet**, daß bei der Deltamodulation, bei der das Vorzeichensignal (VZ) in Abhängigkeit davon erzeugt wird, ob das elektrische Signal (S) ein mit einem vorgegebenen Änderungsbetrag in unterschiedlichen Richtungen änderbares Vorhersagesignal (Prädiktorsignal, A) -über- oder unterschreitet, unterschiedliche Änderungsbeträge für das Vorhersagesignal (A) in Abhängigkeit von dem Vorzeichensignal (VZ) einstellbar sind und daß die Datenwörter (D1-D4) zusätzlich jeweils einen den aktuell eingestellten Änderungsbetrag bezeichnenden Informationsanteil enthalten.

**Claims**

1. Method for processing an electrical signal (S), in particular derived from a heart, whereby the electrical signal (S) is converted by means of delta modulation into a binary sign signal (VZ) consisting of a pulse sequence with the discrete-time logical sign values **zero** or **one**, characterized in that the pulse sequence is converted into four different data words (D1-D4) which are in each case generated one directly after the other in the sequence of the occurrence of four possible states (I-IV), whereby

a first data word (D4) indicates a first state identifier (ZKI) and the length (L4) of a first state (I), where the sign signal (VZ) for a specified first minimum duration (N1) continuously has the logical value **zero**,
a second data word (D3) indicates a second state identifier (ZKII) and the length (L3) of a second state (II), where the sign signal (VZ) for a specified second minimum duration (N2) continuously has the logical value **one**,

a third data word (D1) indicates a third state identifier (ZKIII) and the length (L1) of a third state (III), where the sign value changes within the specified minimum durations (N1, N2) and the sum of all sign values **one** exceeds the sum of all sign values **zero** by a specified first amount (Z1), and

a fourth data word (D2) indicates a fourth state identifier (ZKIV) and the length (L2) of a fourth state (IV), where the sign value changes within the specified minimum durations (N1, N2) and the sum of all sign values **zero** exceeds the sum of all sign values one by a specified second amount (Z2).

2. Method according to claim 1, characterized in that for the first and second minimum duration (N1, N2) the same values are provided.

3. Method according to claim 1 or 2, characterized in that for the first and second amount (Z1, Z2) the same values are provided.

4. Method according to one of the preceding claims, characterized in that to determine the length (L1-L4) of the respective states (I-IV) the number of discrete-time sign values occurring during these states (I-IV) is counted in a counter (13).

5. Method according to one of the preceding claims, characterized in that to determine whether the sum of all sign values **one** exceeds or falls below the sum of all sign values **zero** by the specified amounts (Z1, Z2), the occurrence of the sign values is counted in an additional counter (33) with a counting direction which can be reversed in dependence upon the respective sign values **zero** or **one**, until the count value exceeds or falls below one of the specified amounts (Z1 or Z2).

6. Method according to one of the preceding claims, characterized in that with the delta modulation, where the sign signal (VZ) is generated depending on whether the electrical signal (S) exceeds or falls below a predictor signal A which can be changed in different directions with a specified change amount, different change amounts for the predictor signal (A) can be set in dependence upon the sign signal (VZ), and in that the data words (D1-D4) additionally in each case have an information content which denotes the currently set change amount.

**Revendications**

1. Procédé de traitement d'un signal électrique (S), notamment dérivé d'un coeur, le signal électrique (S) étant converti au moyen d'une modulation delta en un signal de signe binaire (VZ) constitué d'un train d'impulsions ayant les valeurs de signes logiques zéro ou un discrètes dans le temps, caractérisé par le fait que l'on convertit le train d'impulsions en quatre mots de données (D1 à D4) différents qui sont produits respectivement en se succédant immédiatement dans l'ordre d'apparition de quatre états possibles (I à IV),

un premier mot de données (D4) indiquant un premier identificateur d'état (ZKI) et la longueur (L4) d'un premier état (I) dans lequel le signal de signe (VZ) a, pendant une première durée minimale (N1) prédéterminée, continuellement la valeur logique zéro,

un second mot de données (D3) indiquant un second identificateur d'état (ZKII) et la longueur (L3) d'un second état (II) dans lequel le signal de signe (VZ) a, pendant une seconde durée minimale (N2) prédéterminée, continuellement la valeur logique un,

un troisième mot de données (D1) indiquant un troisième identificateur d'état (ZKIII) et la longueur (L1) d'un troisième état (III) dans lequel la valeur de signe change pendant les durées minimales (N1, N2) prédéterminées et la somme de toutes les valeurs de signes un est supérieure d'un premier montant (Z1) prédéterminé à la somme de toutes les valeurs de signes zéro,

et un quatrième mot de données (D2) indiquant un quatrième identificateur d'état (ZKIV) et la longueur (L2) d'un quatrième état (IV) dans lequel la valeur de signe change pendant les durées minimales (N1, N2) prédéterminées et la somme de toutes les valeurs de signes zéro est supérieure d'un second montant (Z2) prédéterminé à la somme de toutes les valeurs de signes un.

2. Procédé selon la revendication 1, caractérisé par le fait que l'on prévoit pour la première et la seconde durée minimale (N1, N2) des valeurs identiques.

3. Procédé selon la revendication 1 ou 2, caractérisé par le fait que l'on prévoit pour le premier et le second montant (Z1, Z2) des valeurs identiques.

4. Procédé selon l'une des revendications précédentes, caractérisé par le fait que, pour déterminer la longueur (L1 à L4) des états (I à IV), on compte dans un compteur (13) le nombre des valeurs de signes discrètes dans le temps qui apparaissent pendant ces états (I à IV).

5. Procédé selon l'une des revendications précédentes, caractérisé par le fait que, pour déterminer si la somme de toutes les valeurs de signes un est supérieure ou inférieure d'un des montants (Z1, Z2) prédéterminés à la somme de toutes les valeurs de signes zéro, on compte l'apparition des valeurs de signes dans un autre compteur (33), avec un sens de comptage pouvant être commuté en fonction des valeurs de signes respectives zéro ou un, jusqu'à ce que la valeur de comptage soit supérieure ou inférieure à l'un des montants (Z1 ou Z2) prédéterminés.

6. Procédé selon l'une des revendications précédentes, caractérisé par le fait que, lors de la modulation delta lors de laquelle le signal de signe (VZ) est produit en fonction du fait que le signal électrique (S) est supérieur ou inférieur à un signal de prédiction (A) modifiable dans des sens différents d'un montant de la modification prédéterminé, des montants de la modification différents peuvent être réglés pour le signal de prédiction (A) en fonction du signal de signe (VZ) et que les mots de données (D1 à D4) contiennent en plus chacun une composante d'information indiquant le montant de la modification courant réglé.

FIG. 1

FIG. 2

FIG. 3

FIG. 4